# EUROPEAN PATENT SPECIFICATION

(11) **EP 4 049 589 B1**
(45) Date of publication and mention of the grant of the patent: **09.10.2024**
(21) Application number: 21792535.3
(22) Date of filing: 29.03.2021
(51) Int. Cl.: A61B 5/00

(54) **SYRINGE-INJECTION-TYPE BRAIN SIGNAL MEASUREMENT AND STIMULATION STRUCTURE, AND SYRINGE INJECTION METHOD THEREFOR**
STRUKTUR IN FORM EINER SPRITZENINJEKTION ZUM MESSEN UND STIMULIEREN VON GEHIRNSIGNALEN UND SPRITZENINJEKTIONSVERFAHREN DAFÜR
STRUCTURE DE MESURE ET DE STIMULATION DE SIGNAL CÉRÉBRAL DE TYPE À INJECTION DE SERINGUE, ET PROCÉDÉ D'INJECTION DE SERINGUE POUR CELLE-CI

(30) Priority: 20.04.2020 KR 20200047425; 26.03.2021 KR 20210039903
(43) Date of publication of application: 31.08.2022
(60) Divisional application: 23155850.3
(73) Proprietor: Gbrain Inc., Incheon 21984 (KR)
(72) Inventor: YANG, Sung Gu, Incheon 22018 (KR); LEE, Sang Won, Incheon 21389 (KR); PARK, Sung Won, Incheon 21938 (KR); AHN, Jong Hyun, Seoul 06005 (KR); CHAE, Young Cheol, Seoul 04148 (KR); KIM, Je Jung, Seoul 03765 (KR)
(74) Representative: Patrade A/S
(86) International application number: PCT/KR2021/003833
(87) International publication number: WO 2021/215682

(56) References cited:
- WO-A1-2018/227165
- WO-A1-2019/140404
- KR-A- 20140 092 238
- KR-B1- 101 301 007
- KR-B1- 101 972 931
- US-A1- 2009 018 630
- US-A1- 2014 094 674
- US-A1- 2018 224 433
- US-A1- 2020 108 246
- PARK SUNG-WON ET AL: "Epidural Electrotherapy for Epilepsy", vol. 14, no. 30, 27 June 2018 (2018-06-27), pages 1801732, XP055859911, ISSN: 1613-6810, Retrieved from the Internet <URL:https://api.wiley.com/onlinelibrary/tdm/v1/articles/10.1002%2Fsmll.201801732> DOI: 10.1002/smll.201801732
- PARK SUNG-WON, KIM JEJUNG, KANG MINPYO, LEE WONHO, PARK BYONG SEO, KIM HANSUNG, CHOI SE-YOUNG, YANG SUNGCHIL, AHN JONG-HYUN, YANG : "Epidural Electrotherapy for Epilepsy", SMALL, vol. 14, no. 30, 1 July 2018 (2018-07-01), pages 1801732, XP055859911, ISSN: 1613-6810, DOI: 10.1002/smll.201801732

## Description

### Technical Field

The present invention relates to a syringe-injection-type brain signal measurement and stimulation structure. Further disclosed is a syringe injection method therefor, which is not part of the present invention.

### Background Art

A brain insertable medical device refers to a device including multiple microelectrodes for obtaining nerve signals or delivering electric stimuli, and plays the role of a nerve interface connecting neurons to electronic circuits. Electric stimuli have less side effects than drug treatment or resection, and have the advantages of reversibility and adjustability.

Currently commercialized brain insertable medical devices are hard and bulky, making it inevitable to open a large portion of the skull when inserted into the brain, and cerebrum-penetrating electrode rods need to be used to stimulate deep parts of the brain. This poses a problem in that the brain pressure is changed by skull opening, deep parts of the brain are severely damaged and infected, and other side effects occur.

In addition, electronic elements inserted into human bodies commonly use soft polymer substrates which are harmless to human bodies mechanically and chemically, and most of them thus have hydrophobic surface characteristics and poorly attach to biological tissues. In order to solve such problems, biocompatible medical adhesives or the like are conventionally used, but this adversely affects the interface between the electronic elements and the biological tissues, making high-quality bio-signal measurement impossible.

US 2009/0018630 A1 disclose a neurostimulation device for inhibitory cortical stimulation of the brain for treating brain disorders or to enhance recovery from brain surgery. A self-expandable epidural cortical electrode having an electrically conductive expandable body, a control unit, and a connecting lead connecting the expandable body by way of a connecting element to the control unit. The flexible body is formed by an open framework defining internal apertures therein, where joints functions as hinges in order to fold, collapse, or bend the electrode.

Park Sung-Wong et al. disclose a graphene electrode for treating epilepsy which can record and pass electrical signals into cortical areas. The graphene is transferred to a SU-8 substrate. The transferred graphene is patterned using photolithography and etching. All nonconductive areas and gold lines are encapsulated with a thin SU-8 epoxy layer.

US 2014/0094674 A1 disclose implantable wireless devices and methods for providing an electrical interface to a body. The implantable module comprises an implantable electrode array, an amplifier circuit, a wireless transceiver, and a sealed enclosure that houses the amplifier circuit and the wireless transmitter.

US 2020/108246 A1 disclose a system for deploying an electrode array at a target location through a hole formed in the patient's cranium. The system includes an array of electrode attached to a substrate and an inserter attached to the substrate and/or the array of electrodes.

US 2018/0224433 A1 disclose electronic devices that can be injected or inserted into soft matter, such as biological tissue. The device, after insertion or injection, may be connected to an external electric circuit.

WO 2018/227165 A1 disclose an injectable electrode which is manufactured in the body by curing from a liquid phase to a solid phase, and therefore molding to the contours of the bodily structure where it is injected.

### Disclosure of Invention

### Technical Problem

In order to solve the above-mentioned problems, the present invention provides a high-performance syringe-injection-type brain signal measurement and stimulation structure according to claim 1. Further aspects of the present invention are defined in the dependent claims. There is further disclosed a syringe injection method therefor, which is not part of the present invention, wherein skull opening can be minimized during insertion into the brain, and the structure has excellent attachment to biological tissues.

### Solution to Problem

To solve the technical problems, the disclosure provides a brain signal measurement and stimulation structure including: a flexible element including a contact part configured to be in contact with the surface of a cerebral cortex to measure a signal generated in the brain or transmit an external stimulus to the brain, a transmitting/receiving part positioned between a skull and the skin, and a connection part configured to connect the contact part and the transmitting/receiving part; and
an integrated circuit connected to the transmitting/receiving part to transmit and receive a signal, wherein
the flexible element includes a lower-layer support substrate, a graphene electrode layer and a wiring layer formed on the lower-layer support substrate, and an insulation layer formed on the graphene electrode layer and the wiring layer, the insulation layer is etched such that the graphene electrode layer is partially exposed, and a part of the graphene electrode layer and a part of the wiring layer are adjacently connected.

The flexible element has a serpentine structure.

In addition, a surface of the insulation layer undergoes hydrophilic surface treatment. Furthermore, a partial surface of the lower-layer support substrate undergoes hydrophobic surface treatment. The hydrophilic surface treatment may be performed by spin-coating the insulation layer, masking the exposed graphene electrode layer, and then treating with O₂ or O₃. In addition, the hydrophobic surface treatment may be performed such that a hydrophobic microstructure including a plurality of microprotrusions is formed.

The integrated circuit may include a wireless power supply device, a recording device, a stimulation device, and a communication device.

The lower-layer support substrate may be one selected from the group consisting of polyethylene terephthalate (PET), polyimide (PI), polystyrene (PS), polycarbonate (PC), polyethersulfone (PES), polymethylmethacrylate (PMMA) cyclo-olefin polymers (COP), polydimethylsiloxane (PDMS), polyvinylpyrrolidone (PVP), polyethylene naphthalate (PEN), polyvinyl chloride (PVC), and a mixture thereof. In addition, the graphene electrode layer includes one to four layers. The graphene electrode layer may have a diameter of 30 to 150 µm.

The wiring layer may be formed of one selected from the group consisting of gold (Au), silver (Ag), copper (Cu), nickel (Ni), and iron (Fe). In addition, the wiring layer may have a thickness of 30 to 60 nm.

The insulation layer may be formed of one selected from the group consisting of optical clean resin (OCR), optical clean adhesive (OCA), SU-8, and a mixture thereof.

A syringe injection method for a brain signal measurement and stimulation structure of the disclosure,
including a flexible element including a contact part configured to measure a signal generated in the brain or transmit an external stimulus to the brain, a transmitting/receiving part configured to be connected to an integrated circuit, and a connection part configured to connect the contact part and the transmitting/receiving part, includes:
opening a scalp, forming a small hole in the cranial bone, and inserting a thin tube into which a syringe can be inserted to connect the brain to the outside;
inserting an outlet of a syringe containing the flexible element through the tube;
applying pressure to the syringe to insert the flexible element and a cerebrospinal fluid into the brain through the outlet; and
bringing the contact part of the flexible element into contact with the cerebral cortex, positioning the transmitting/receiving part between the skull and the skin so as to be connected to the integrated circuit, and penetrating the skull by the connection part so as to connect the contact part and the transmitting/receiving part.

A brain signal measurement and stimulation structure of the disclosure may include: a flexible element including a contact part configured to be in contact with the surface of the cerebral cortex to measure a signal generated in the brain or transmit an external stimulus to the brain, a transmitting/receiving part positioned between a skull and a skin, and a connection part configured to connect the contact part and the transmitting/receiving part; and
an integrated circuit connected to the transmitting/receiving part to transmit and receive a signal,
wherein the contact part may have a surface configured such that, when a pressure is applied to a syringe containing the flexible element so as to inject the flexible element into the brain, the surface receives the most pressure from a fluid included in the syringe, and may be unfolded by the flow of the fluid.

The connection part is formed in a spiral shape between the contact part and the transmitting/receiving part to have a three-dimensional structure.

### Advantageous Effects of Invention

The disclosure provides a brain signal measurement and stimulation structure including a flexible element and an integrated circuit, and the flexible element of the disclosure has excellent flexibility and mechanical characteristics such that the same can be injected only by opening the skull in a very small area and can function as a high-performance element for measuring brain signals and providing electric stimuli.

### Brief Description of Drawings

FIG. 1 illustrates a configuration of a flexible element according to an embodiment of the disclosure.
FIG. 2 illustrates a syringe-injection-type brain signal measurement and stimulation structure (a flexible element and an integrated circuit) injected into the brain and attached thereto, according to an embodiment of the disclosure.
FIG. 3 illustrates an integrated circuit attached to the skull surface, according to an embodiment of the disclosure.
FIG. 4 schematically shows an internal configuration of an integrated circuit according to an embodiment of the disclosure.
FIG. 5 is a view for explaining a method for injecting a syringe into the brain and mounting a brain signal measurement and stimulation structure in the brain, according to an embodiment of the disclosure.
FIG. 6 illustrates a cross-section of a contact part according to an embodiment of the disclosure.
FIG. 7 illustrates a cross-section of a contact part according to another embodiment of the disclosure.
FIG. 8 illustrates a pattern of an insulation layer of a contact part according to an embodiment of the disclosure.
FIG. 9 illustrates a pattern of a lower-layer support substrate of a contact part according to an embodiment of the disclosure.
FIG. 10 is an enlarged view of a connection part of a flexible element according to an embodiment of the disclosure.
FIG. 11 is a view for explaining the characteristics of the serpentine structure of a flexible element according to an embodiment of the disclosure.
FIG. 12 shows the result of measuring the resistance change due to the tensile deformation of a flexible element according to an embodiment of the disclosure.
FIG. 13 shows a method for manufacturing a flexible element according to another embodiment of the disclosure.
FIG. 14 shows the thickness and diameter of each layer of a flexible element according to another embodiment of the disclosure.
FIG. 15 illustrates a configuration of a contact part of a flexible element according to another embodiment of the disclosure.
FIG. 16 illustrates a configuration of a connection part and transmitting/receiving part of a flexible element according to another embodiment of the disclosure.
FIG. 17 illustrates a connection relationship between a side view of a flexible element and a plan view of the flexible element according to another embodiment of the disclosure.
FIG. 18 illustrates a spiral shape of a connection part according to another embodiment of the disclosure.
FIG. 19 illustrates a syringe in which a flexible element is accommodated, according to another embodiment of the disclosure.
FIG. 20 shows a structural concept of a flexible element according to another embodiment of the disclosure.

### Best Mode for Carrying out the Invention

Throughout the specification, when a part "includes" a certain element, it means that other elements may be further included, rather than excluding other elements, unless otherwise stated.

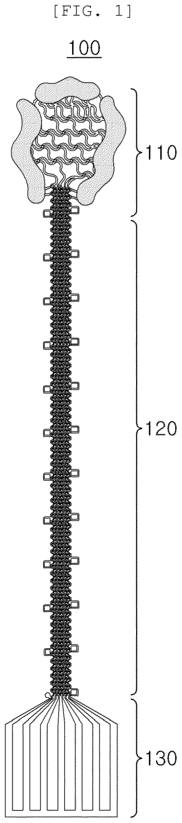

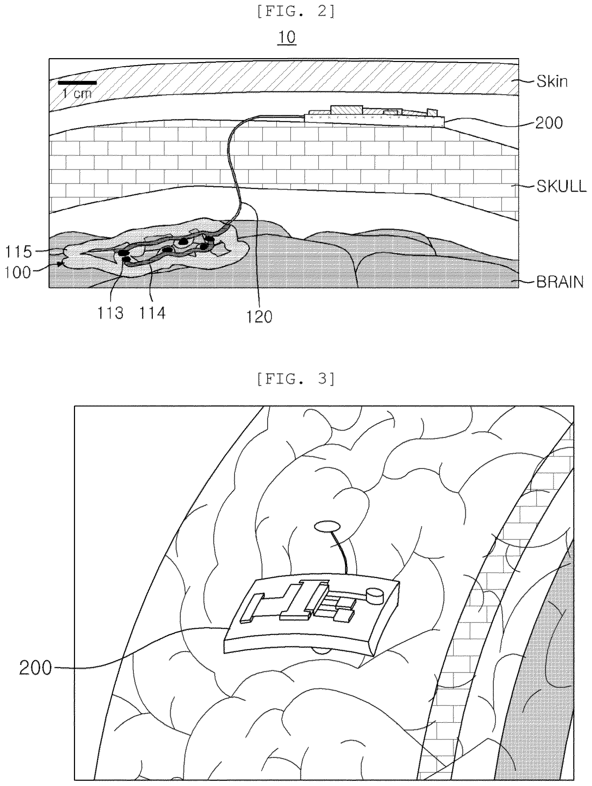

FIG. 1 illustrates a configuration of a flexible element according to an embodiment of the disclosure, FIG. 2 illustrates a syringe-injection-type brain signal measurement and stimulation structure (a flexible element and an integrated circuit) injected into the brain and attached thereto, according to an embodiment of the disclosure, and FIG. 3 illustrates an integrated circuit attached to the skull surface, according to an embodiment of the disclosure.

A syringe-injection-type brain signal measurement and stimulation structure 10 according to an embodiment of the disclosure includes a flexible element 100 and an integrated circuit 200.

The flexible element 100 of the embodiment of the disclosure includes a contact part 110, a connection part 120, and a transmitting/receiving part 130. Specifically, the flexible element 100 includes the contact part 110 in contact with the surface of cerebral cortex, the transmitting/receiving part 130 installed in a space between a skull and a skin, and the connection part 120 connected to the end of the contact part 110 and the end of transmitting/receiving part 130 and disposed inside and outside the skull while passing through the skull.

Here, the contact part 110 is in contact with the cerebral cortex to serve to measure a signal generated in the brain or to transmit an external stimulus to the brain.

FIG. 2 shows that the flexible element 100 is injected into the brain by using a syringe, the contact part 110 is in contact with the cerebral cortex, and the transmitting/receiving part 130 of the flexible element 100 is connected to the integrated circuit 200 to be in contact with the skull surface.

The brain signal measurement and stimulation structure 10 of the disclosure may include a flexible electrode, wireless link, and an element for a wireless power supply, recording, and stimulation.

Among the flexible elements 100 injected into the brain, the contact part 110 is in contact with the cerebral cortex, and the connection part 120 penetrates the skull to connect the contact part 110 and the transmitting/receiving part 130.

The transmitting/receiving part 130 is coupled to the integrated circuit 200 and is positioned between the skull and the skin. The transmitting/receiving part 130 is attached to the skull and is coupled to the integrated circuit 200 shown in FIG. 3. FIG. 3 schematically illustrates an integrated circuit 200 attached to the skull surface.

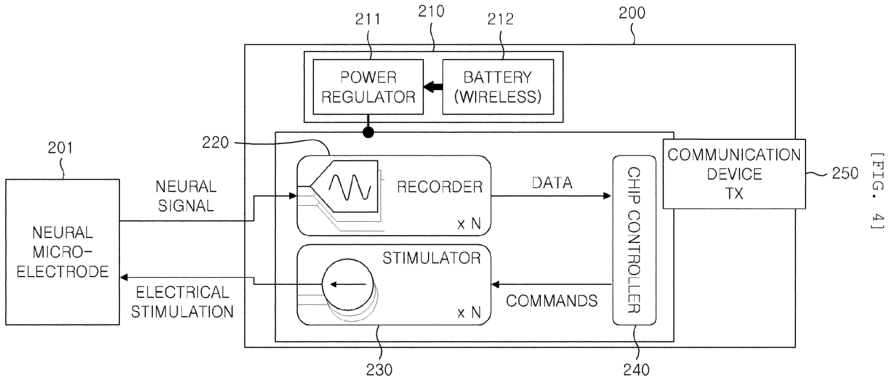

FIG. 4 schematically shows an internal configuration of an integrated circuit according to an embodiment of the disclosure.

The integrated circuit 200 of the embodiment of the disclosure may be a wireless chip, and serves to transmit and receive radio waves. The integrated circuit 200 may include a wireless power supply device, a recording device, a stimulation device, and a communication device, and may be a package including the above-described elements and capable of enhancing biocompatibility.

The integrated circuit 200 is installed in a space between the skull and the skin. Specifically, the wireless power supply device is for supplying power to the integrated circuit 200, the recording device is for recording the measured brainwaves, the stimulation device is for providing a stimulus to the brain, and the communication device is an element for transmitting the measured brainwaves to the outside and receiving external commands.

Referring to FIG. 4, the integrated circuit 200 records radio wave measurement value received from the brain to obtain biometric information so as to detect an abnormal signal. When an abnormality is detected, the integrated circuit 200 applies energy such as electric current, voltage, magnetic field, or electric field stimulus to perform neuro-modulation, thereby serving to treat diseases or control brain activity.

The integrated circuit 200 includes a wireless chip power supply part 210, which is a wireless power supply device, a recorder 220, which is a recording device, a stimulator 230, which is a stimulation device, a chip controller 240, and a communication device 250.

The wireless chip power supply part 210 includes a power regulator 211 and a battery 212.

The battery 212 may perform wireless energy harvesting by using an RF coil or the like, and may store energy supplied in a wireless manner.

The battery 212 may be charged in a wireless manner using a wireless power transfer (WPT) technology.

The power regulator 211 converts AC of the battery 212 into DC and transmits the DC to the chip controller 240.

A neural micro electrode 201 is an electrode attached to the brain and enables brainwave measurement and electrical stimulation.

The recorder 220 is used for the brainwave measurement and converts analog data into digital data to transmit the data to the chip controller 240, and the data is wirelessly transmitted to an external device through the communication device 250 from the chip controller 240. The recorder 220 varies according to the number of brainwave measurement panels.

The recorder 220 may perform input of multi-channel brainwave measurement data and digital signal conversion.

The chip controller 240 may control a measurement signal and a stimulation signal.

The communication device 250 is wirelessly connected to an external communication network to enable monitoring outside the body. The communication device 250 may wirelessly transmit the digital signal converted by the recorder 220 through an electrode or an antenna.

As for the integrated circuit 200, in consideration of problems such as packaging heat generation, it is necessary that a package material of the integrated circuit is compatible with a living body to ensure biocompatibility and does not affect a chip during encapsulation formation.

As for the integrated circuit 200, any material generally used in a living body in the field may be used as a package material without limitation, but polydimethylsiloxane (PDMS), parylene C, polyimide, biocompatible UV resin, etc. are suitable.

Specifically, when the contact part 110 of the flexible element 100 measures brainwaves through a graphene electrode layer 113 exposed to the outside to transmit the same to the integrated circuit 200, the brainwaves may be wirelessly transmitted to the outside through the integrated circuit 200.

When an abnormality is detected in the brainwave signal measured by the chip controller 240, the brainwave signal is transmitted to the outside through the communication device 250, and a command is transmitted to the stimulator 230 to apply an electrical stimulation signal to the neural micro electrode 201. The stimulator 230 varies according to the number of electrical stimulation channels. The stimulator 230 may generate or deliver a therapeutic stimulus commanded to the chip controller 240.

Referring to FIG. 4, the brainwaves measured through the contact part 110 of the flexible element 100 may be converted into a digital signal through the recorder 220 and transmitted to the integrated circuit 200, which may be wirelessly transmitted a smart device, such as a smartphone and a smart pad, a computer, or the like. The one or more recorders 220 may be included.

Radio waves received from the outside through the integrated circuit 200 may be transmitted to the brain through the flexible element 100.

Through the process described above, when an abnormality is detected in the brainwaves measured by the contact part 110, the brainwaves may be transmitted to the outside, and the integrated circuit 200 may be controlled to provide an electrical signal to the contact part 110. For example, referring to FIG. 4, the integrated circuit 200 may transmit a command to the stimulator 230 to stimulate the cerebral cortex. The one or more stimulators 230 may be included.

On the other hand, as will be described later, the integrated circuit 200 may cause external radio waves received by a separate wireless communication device to be transmitted to the integrated circuit through an electrode or an antenna, and thus may also serve to transmit the external radio waves to the brain without receiving the external radio waves as they are.

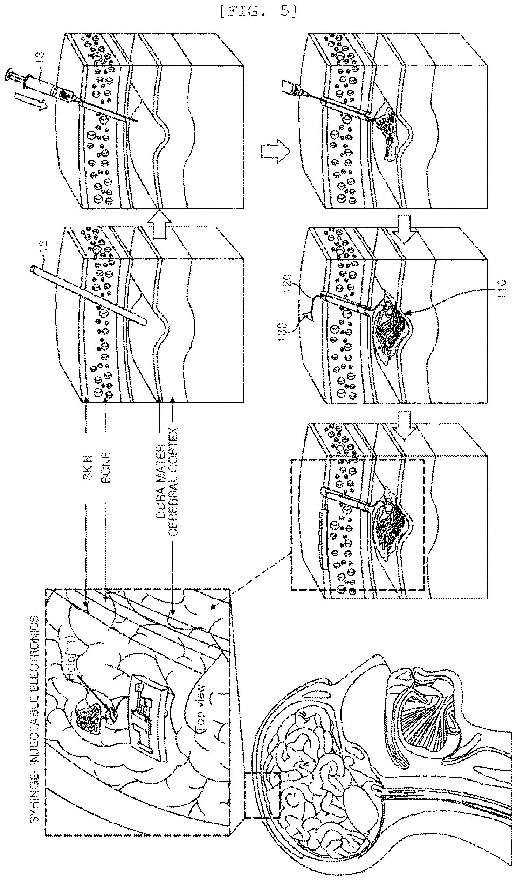

FIG. 5 is a view for explaining a method for injecting a syringe into the brain and mounting a brain signal measurement and stimulation structure in the brain, according to an embodiment of the disclosure.

The method for injecting the flexible element 100 into the brain by using a syringe 13 will be described as follows.

First, a scalp is opened, a small hole 11 is formed in a cranial bone, and a thin tube 12 into which the syringe 13 can be inserted is inserted to connect the brain to the outside.

The outlet of the syringe 13 containing the flexible element 100 is inserted through the inserted tube 12 without damaging the brain.

The flexible element 100 and the cerebrospinal fluid are inserted into the brain through the outlet by applying pressure to the syringe 13.

The contact part 110 of the flexible element 100 is brought into contact with the cerebral cortex, the transmitting/receiving part 130 is positioned between the skull and the skin, and the connection part 120 penetrates the skull and connects the contact part 110 and the transmitting/receiving part 130.

The pressure of the syringe 13 is adjusted such that the contact part 110 is positioned on the cerebral cortex, and the transmitting/receiving part 130 is positioned between the skull and the skin.

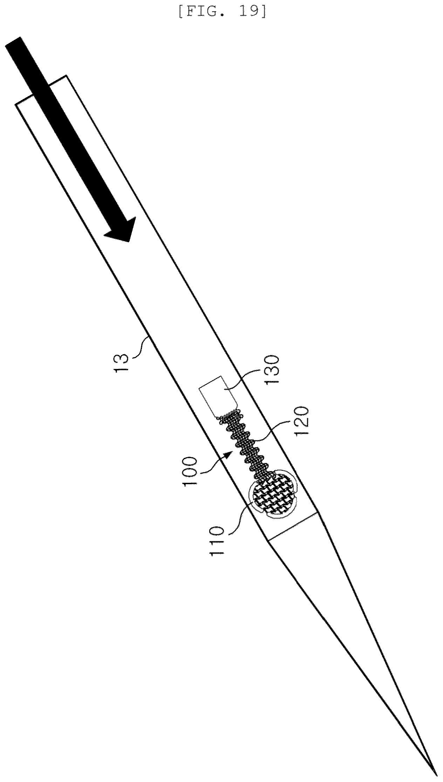

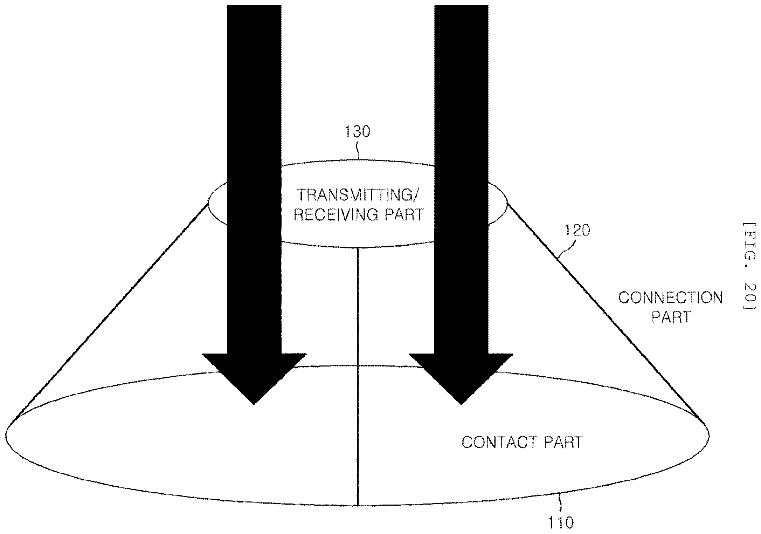

The transmitting/receiving part 130 of the flexible element 100 positioned on the cranial bone is connected to the integrated circuit 200. A process performed inside the skull by the flexible element 100 injected into the brain by the syringe 13 in a process of injecting the syringe 13 in FIG. 5 will be described below in detail with reference to FIGS. 19 and 20.

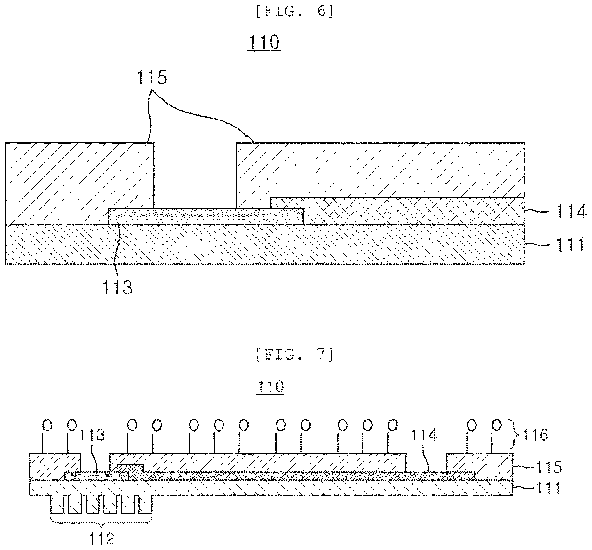

FIG. 6 illustrates a cross-section of a contact part according to an embodiment of the disclosure, and FIG. 7 illustrates a cross-section of a contact part according to another embodiment of the disclosure.

As shown in FIG. 6, the contact part 110 according to the embodiment of the disclosure includes a lower-layer support substrate 111, a graphene electrode layer 113 and a wiring layer 114 which are formed on the lower-layer support substrate 111, and an insulation layer 115 formed on the graphene electrode layer 113 and the wiring layer 114.

The insulation layer 115 is etched such that a part of the graphene electrode layer 113 is exposed, and a part of the graphene electrode layer 113 and a part of the wiring layer 114 are adjacently connected.

As shown in FIG. 7, the disclosure may include an additional element provided to improve the adhesion of the contact part 110 to the wet biological tissue. The additional element includes a hydrophilic functional group 116 bonded to the surface of the insulation layer 115 by hydrophilic surface treatment, and a hydrophobic microstructure 112 formed on a partial surface of the lower-layer support substrate 111 to be opposite to the hydrophilic functional group 116.

The insulation layer 115 is etched such that a part of the wiring layer 114 is exposed.

The reason for the hydrophilic surface treatment is that the flexible element 100 is a syringe-injection type. Accordingly, as the flexible element 100 is pushed into the brain, the hydrophilic surface-treated insulation layer 115 is attached to the surface of the cerebral cortex due to the adhesion between the hydrophilic surfaces. Therefore, the front and back surfaces of the flexible element 100 are prevented from being reversed, and the flexible element 100 is prevented from being folded.

The hydrophobic microstructure 112 may include a plurality of microprotrusions having hydrophobicity to improve adhesion of living tissue.

FIG. 7 shows a cross-section of the contact part 110 having the insulation layer 115 and the lower-layer support substrate 111, both of which are surface-treated.

Referring to FIG. 7, the surface characteristics opposite to each other may be formed on the surfaces of the insulation layer 115 at the upper end of the flexible element and the lower-layer support substrate 111 at the lower end, respectively, to improve adhesion. The hydrophilic functional group 116 formed through the hydrophilic surface treatment applied to the surface of the insulation layer 115 may improve adhesion by enabling interaction with cerebrospinal fluid (brain water).

A partial surface of the lower-layer support substrate 111 undergoes a hydrophobic surface treatment.

The hydrophobic microstructure 112 additionally formed on the lower-layer support substrate 111 may include innumerable protrusion-shaped structures to maximize hydrophobic properties so as to improve adhesion.

The lower-layer support substrate 111 may be generally hydrophobic, and the hydrophobic surface property may be maximized through the hydrophobic surface structure formation to improve adhesion to the body.

As will be described later, the hydrophobic surface treatment may be performed simultaneously with the formation of the lower-layer support substrate 111, by etching a sacrificial layer to form a pattern on a Si/SiO₂ wafer and then spin-coating a material, serving as a material for the lower-layer support substrate, onto SiO₂.

The contact part 110 of the disclosure has a strong contact force due to the very thin total thickness thereof, and is easily contacted with the surface of the cerebral cortex due to the small bending stiffness thereof. In addition, since the contact part 110 has considerable mechanical flexibility by employing a serpentine mesh structure, performance thereof is not degraded even when the injection into the brain is performed through a small-diameter glass pipet.

The disclosure provides a method for measuring a brain signal by using the flexible element 100 and a method for stimulating the brain.

The contact part 110 may receive an electrical signal and the like generated in the brain through contact with the cerebral cortex. Specifically, the graphene electrode layer 113 of the contact part 110 of the flexible element 100 serves to measure an electrical signal. The electrical signal may be transmitted to the integrated circuit 200 connected to the transmitting/receiving part 130 through the wiring layer 114, and thus the received brain signal may be visually provided through a terminal as will be described later.

Meanwhile, the transmitting/receiving part 130 may be positioned in a space between the skull and the skin to receive an external stimulus and deliver the same to the cerebral cortex. Specifically, the transmitting/receiving part 130 may receive an external stimulus and transmit the same to the cerebral cortex.

As an example, the transmitting/receiving part 130 may be connected to the integrated circuit 200, and the integrated circuit may be in contact with the skull surface.

The lower-layer support substrate 111 serves as a support substrate for supporting a graphene electrode, and is made of a material having flexible properties in order to achieve the objective of the disclosure. In addition, since only the open graphene electrode part of the element should be attached to the living tissue, the lower-layer support substrate is preferably an insulator.

Accordingly, the lower-layer support substrate may be one selected from the group consisting of polyethylene terephthalate (PET), polyimide (PI), polycarbonate (PC), polyethersulfone (PES), polydimethylsiloxane (PDMS), polyvinylpyrrolidone (PVP), polyethylene naphthalate (PEN), polyvinyl chloride (PVC), and a mixture thereof, but is not limited thereto. In the element of the disclosure, the material of the lower-layer support substrate may employ polyimide (PI) in terms of excellent chemical resistance to various chemicals used in the manufacturing process and excellent mechanical properties that can withstand mechanical deformation even at a thickness of several µm.

The lower-layer support substrate 111 may have a thickness of 0.5 to 2 um in terms of considering the flexibility of the contact part 110 and the buckling effect that occurs in the serpentine structure when mechanical deformation is applied thereto and optimizing the effective bending stiffness of the entire structure of the element. However, the disclosure is not limited thereto, and may be changed according to conditions such as a structure of an element or a material used by those skilled in the art.

In the disclosure, the graphene electrode layer 113 is a part which directly performs measurement. The graphene electrode layer 113 of the disclosure may include a single layer, but may include one to four layers. In case that the graphene electrode 113 has a multi-layer structure, the conductivity of graphene increases and impedance decreases, and when mechanical deformation is applied thereto, cracks occurring in each layer of graphene are buffered by each layer, and thus the graphene electrode layer 113 can further withstand deformation.

Considering that the signal to noise ratio (SNR) is dominantly changed by the impedance that changes according to the spatial geometry of an electrode when the graphene electrode layer 113 has a diameter of 30 to 150 µm, the graphene electrode layer 113 may have a diameter of 30 um or more. Considering the minimum resolution by synchronization of brain signals measured on the brain surface, the graphene electrode layer 113 may have a diameter of 150 um or less. However, the disclosure is not limited thereto, and the diameter may be changed according to conditions by those skilled in the art.

In addition, the graphene electrode layer 113, which is a sensing part of the contact part 110 of the disclosure, may be positioned on a neutral mechanical plane (NMP). That is, since the graphene electrode layer 113 serves to measure an electrical signal of a living tissue, the graphene electrode layer 113 may be positioned on the NMP to minimize mechanical deformation thereof.

In the disclosure, the wiring layer 114 may be formed of one selected from the group consisting of gold (Au), silver (Ag), copper (Cu), nickel (Ni), and iron (Fe).

The wiring layer 114 may have a thickness of 30 to 60 nm because the wiring layer 114 may be less susceptible to cracks that may be caused by twist due to the mechanical deformation in the serpentine structure. However, the disclosure is not limited thereto, and the thickness may be changed according to conditions by those skilled in the art.

In the disclosure, the insulation layer 115 may be formed of one selected from the group consisting of optical clean resin (OCR), optical clean adhesive (OCA), SU-8, and a mixture thereof, but is not limited thereto. The material that is non-conductive and can be used for passivation to prevent crosstalk may be used as the insulation layer 115 of the disclosure without limitation.

Since the insulation layer 115, which is an upper polymer layer, is required to be formed such that the graphene electrode part thereof is opened, the insulation layer should be patternable without undergoing a dry etching process such as RIE which may damage the graphene electrode. Therefore, an SU-8, which is a type of photoresist, may be used for the insulation layer. The upper SU-8 layer may be patterned using a photo process to form a serpentine network structure according to the shape of the lower layer.

The insulation layer 115 may be etched such that the graphene electrode layer 113 is exposed, and may be divided into a first insulation layer which is a part not in contact with the wiring layer 114 and a second insulation layer which is a part in contact with the wiring layer 114. Specifically, the second insulation layer is in contact with both the graphene electrode layer 113 and the wiring layer 114, and the first insulation layer is in contact with the lower-layer support substrate 111 and the graphene electrode layer 113.

The insulation layer 115 may have a thickness of 0.5 to 2 um as a thickness for enabling a sensing part on a neutral mechanical plane (NMP). However, the disclosure is not limited thereto, and the thickness may be changed according to conditions by those skilled in the art.

Meanwhile, as described above, the disclosure may further include an element to improve the adhesion of the flexible element 100 to the body.

The process of improving the adhesion of the flexible element 100 of the disclosure to the body is as follows.

A pattern is formed on a Si/SiO₂ wafer by etching a sacrificial layer. When the hydrophobic surface treatment is not performed on the lower-layer support substrate, the SiO₂ etching process may be omitted.

Thereafter, the lower-layer support substrate 111 is formed by spin-coating on SiO₂. After the graphene electrode layer 113 is formed on the lower-layer support substrate 111 by graphene transfer, photolithography, and dry etching processes, the wiring layer 114 is laminated and patterned.

Then, the insulation layer 115 is spin-coated, the exposed graphene electrode layer 113 is subjected to masking, and then the hydrophilic surface treatment is performed. In this case, the masking is to prevent damage to the graphene, and is performed capping the exposed graphene by using a photoresist material 30, but is not limited thereto. In addition, the hydrophilic surface treatment is performed using O₂ or O₃ treatment, but may be performed by a method generally available to those skilled in the art. Specifically, the surface treatment may be performed using O₂ plasma or UV ozone.

Thereafter, after the hydrophilic surface treatment, the masking material is removed, and the SiO₂ layer is etched, thereby preparing a flexible element of the disclosure which has undergone the hydrophilic and hydrophobic surface treatment.

Meanwhile, the brain signal measurement and stimulation structure 10 of the disclosure may further include an amplifier. The amplifier may amplify the electrical signal measured at the graphene electrode of the flexible element 100 of the disclosure.

The structure 10 of the disclosure has been described that the same is injected into the brain. However, the position at which the structure 10 is injected may vary within the range applicable to those skilled in the art, and may be inserted or implanted into the spinal cord, peripheral nerves, etc.

The syringe-injection-type brain signal measurement and stimulation structure 10 of the disclosure may be used in vitro, ex vivo, or in vivo. The living body may an animal and may include a human, but may also be an animal other than a human.

The disclosure provides a brain implantable medical device including the syringe-injection-type brain signal measurement and stimulation structure 10. The medical device of the disclosure includes an integrated circuit 200 connected to the transmitting/receiving part 130 of the flexible element 100. Specifically, the injection of the flexible element 100 may be achieved with only several mm² skull area opening, the contact part 110 of the flexible element 100 may be in contact with the cerebral cortex, the transmitting/receiving part 130 may be positioned between the skull and the skin, and the connection part 120 configured to connect the contact part 110 and the transmitting/receiving part 130 may be installed by penetrating the skull.

In addition, the integrated circuit 200 may be connected to the transmitting/receiving part 130 of the flexible element 100 and may be installed between the skull and the skin, and preferably may be in contact with the skull surface.

Furthermore, the disclosure provides a brain signal measurement and stimulation module including the brain signal measurement and stimulation structure 10 and further including a wireless communication device configured to transmit external radio waves to the integrated circuit 200. That is, as described above, transmission and reception may be performed only using a wireless module such as Bluetooth or wireless communication network (WiFi) within the flexible element 100 and the integrated circuit 200, but an additional wireless communication device may be used for safer signal transmission and reception through body channel communication (BCC).

Specifically, the external radio waves received through the wireless communication device is transmitted to the integrated circuit through a BCC antenna. Through this, the integrated circuit serves to the external radio waves to the brain through the BCC antenna without receiving the external radio waves as they are.

Any device capable of receiving an external stimulus and transmitting the same to an integrated circuit may be used as the wireless communication device without limitation, and the wireless communication device may be a device which is to be in close contact with the skin. For example, the wireless communication device may be a smart device worn on a wrist or waist.

The wireless communication device may communicate with an external terminal, such as a computer and a smartphone, through Bluetooth and short-range wireless networks (Wi-Fi), etc., to provide various information to a user through the measured brainwave signals, and may transmit the stimulus transmitted from a terminal to the integrated circuit. Additionally, when an emergency signal is generated, the wireless communication device may also be provided with a function for transmitting information to a medical institution.

The disclosure relates to a brain signal measuring method for receiving a brain signal by injecting the syringe-injection-type brain signal measurement and stimulation structure 10 into the brain of an animal. Animals may refer to animals excluding humans. The disclosure can measure a brain signal through the contact part 110 of the flexible element 100 which is in contact with the cerebral cortex.

In addition, the disclosure relates to a method for receiving a brain signal of an animal through the structure 10 and providing the received information to the outside of the body. Animals may refer to animals excluding humans. For example, the disclosure may further include an interface configured to transmit a brain signal which is measured by the flexible element 100 and is received by the integrated circuit, to a user terminal.

In this case, the signal amplified by the amplifier may be transmitted.

Any device capable of visually providing the detected brain signal may be used as the terminal without limitation, and a device capable of acoustically providing the signal may also be combined therewith. As an example, other devices having a screen such as a smartphone or a computer may be used, and in a specific case, information may be provided through a speaker or the like.

The disclosure also provides a method for stimulating the brain by injecting the structure 10 into the brain of an animal. Animals may refer to animals excluding humans. As described above, when a stimulus command is transmitted from the outside, the same may be transmitted to the integrated circuit through an electrode or an antenna, and the stimulus may be provided to the cerebral cortex through the flexible element of the disclosure.

For example, brain stimulation may be achieved through the following process. When an abnormality, such as a seizure, is detected in the brainwaves measured through the flexible element, the integrated circuit receives the brainwaves and provides the same to the outside. When it is determined through the provided information that it is necessary to apply a stimulus from the outside, a stimulus may be transmitted to the integrated circuit and then be transmitted through a stimulator or the like within the integrated circuit to the cerebral cortex. Alternatively, radio waves may be transmitted to a separate wireless communication device and be transmitted to an integrated circuit through an electrode or an antenna, and then be transmitted to the cerebral cortex through the flexible element of the disclosure. Abnormal brainwave signals may be removed through the process described above.

The disclosure is to provide a stimulus to the cerebral cortex through the flexible element 100. The stimulus may be one or more selected from current stimulus, voltage stimulus, electric field stimulus, and magnetic field stimulus, but is not limited thereto.

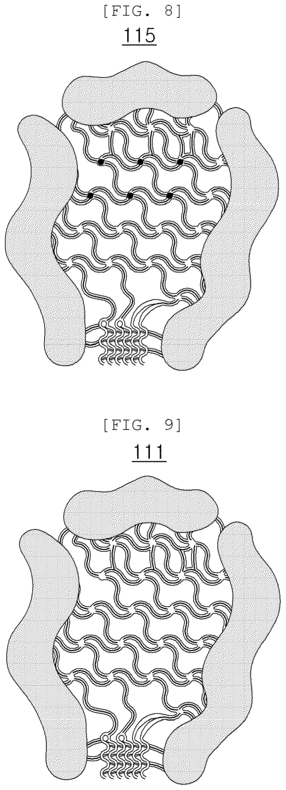

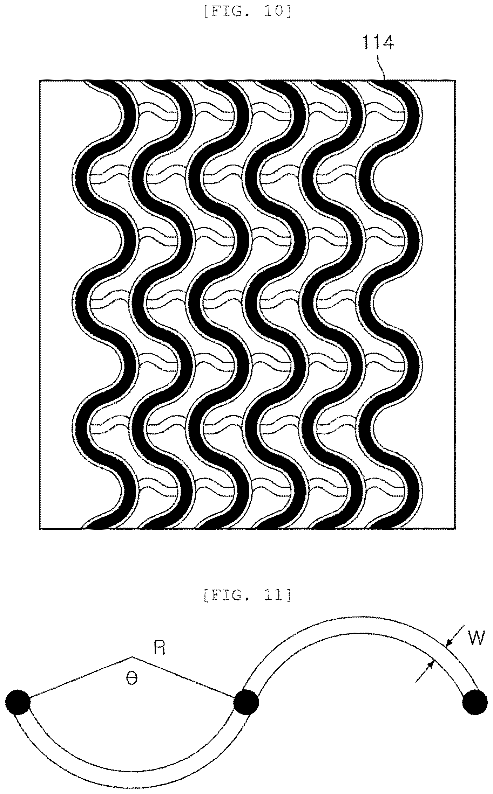

FIG. 8 illustrates a pattern of an insulation layer of a contact part according to an embodiment of the disclosure, FIG. 9 illustrates a pattern of a lower-layer support substrate of a contact part according to an embodiment of the disclosure, FIG. 10 is an enlarged view of a connection part of a flexible element according to an embodiment of the disclosure, and FIG. 11 is a view for explaining the characteristics of the serpentine structure of a flexible element according to an embodiment of the disclosure.

The contact part 110 includes a graphene electrode layer 113 including graphene. Graphene has very low detection sensitivity due to the low electronic noise thereof, and has excellent electrical, mechanical, and thermal properties. The graphene electrode layer 113 is partially exposed by the etched insulation layer.

Since the contact part 110 exhibits considerable mechanical flexibility due to the serpentine structure thereof, the performance thereof is not degraded even when injection into the brain is performed through a glass pipet having a small diameter.

An enlarged configuration of the contact part 110 is shown in FIG. 8. As shown in FIG. 8, the contact part 110 may include an outermost pad and a polymer layer having a serpentine shape and formed therein.

Since the contact part 110 is mechanically flexible due to the inner part thereof having a serpentine shape, the injection thereof may be performed through the skull with a very small open area. The polymer layer formed inside is formed such that the wiring layer 114 is connected to the graphene electrode layer 113 stacked on the lower-layer support substrate 111 and insulation layer 115 surrounds the wiring layer 114 to prevent cross talk.

FIG. 8 specifically illustrates a pattern of the insulation layer 115 which is an upper polymer layer of the contact part 110.

FIG. 9 illustrates a pattern of the lower-layer support substrate 111 which is a lower polymer layer of the contact part 110. FIG. 8 illustrating a pattern of the upper polymer layer is the same as FIG. 9 except that a part electrically connected to the outside due to the open parts of the graphene electrode layer 113 and the wiring layer 114 connected thereto is expressed therein.

In addition, the outermost pad of the contact part 110 may have an additional graphene electrode layer 113 on top thereof, which may be used for the purpose of effectively providing an electrical stimulus by making a wide partial contact with the cerebral cortex.

Since the insulation layer 115, which is an upper polymer layer of the contact part 110, is required to be formed such that the graphene electrode part thereof is opened, the insulation layer should be patternable without undergoing a dry etching process such as reactive ion etching (RIE) which may damage the graphene electrode layer 113.

Therefore, an SU-8, which is a type of photoresist, is generally used. The upper SU-8 layer may be patterned using a photo process to form a serpentine network structure according to the shape of the lower layer.

The transmitting/receiving part 130 may be disposed in a space between the skull and the skin to be connected to the integrated circuit 200 for reception of a measured signal and transmission of stimulus. In particular, the transmitting/receiving part 130 may be fixed in contact with the skull surface. The integrated circuit 200 may receive brainwaves of the cerebral cortex region measured through the contact part 110 and transmit the same to the outside, or may receive an external electrical stimulus to provide the same to the contact part 110.

The connection means that the transmitting/receiving part 130 and the integrated circuit 200 of the flexible element 100 are electrically and physically connected to each other. For example, a wire of the integrated circuit 200 may be connected to each element such as a channel of the transmitting/receiving part 130.

The connection part 120 connects the end of the contact part 110 and the end of the transmitting/receiving part 130 and exhibits considerable mechanical flexibility due to the plurality of wires and polymers thereof having a serpentine mesh structure. Therefore, performance thereof may not be degraded even when the injection into the brain is performed through a small-diameter glass pipet, and the contact part 110 and the transmitting/receiving part 130 may be connected along a fine open area of the skull.

The connection part 120 may have a serpentine network structure by coating a polymer on a metal material constituting the wiring layer 114 to form a cross-link bond.

As an example, the enlarged configuration of the connection part 120 is shown in FIG. 10. Each wiring layer 114 in the connection part 120 is surrounded by the lower-layer support substrate 111 and the insulation layer 115.

Unlike the contact part 110 in which the patterns of the lower-layer support substrate 111 (FIG. 9) and the insulation layer 115 (FIG. 8) are different from each other, in the connection part 120, the patterns of the lower-layer support substrate 111 and the insulation layer 115 are identical to each other.

The transmitting/receiving part 130 is formed by forming a wiring layer 114 on the lower-layer support substrate 111, forming an insulation layer 115 thereon, and then opening the insulation layer 115 at an area of a wiring layer 114 to be connected to the integrated circuit 200.

As shown in FIG. 10, the connection part 120 may penetrate the skull due to the serpentine network structure thereof and connect the contact part 110 in contact with the cerebral cortex and the transmitting/receiving part 130 installed in a space between the skull and the skin.

In the disclosure, the serpentine structure is formed through a patterning process. Specifically, in the case of the lower-layer support substrate 111, crosslinking is performed at a high temperature after a corresponding polymer layer is coated, followed by applying a photoresist on the polymer layer, and then a pattern is formed by a photolithography process. Thereafter, a pattern having a serpentine structure may be formed by etching according to the pattern through a RIE process or the like.

The photoresist may employ the 40xt, but is not limited thereto, and after a pattern is formed through a process such as RIE etching, acetone or the like may be added to strip off the same.

The angle, radius, and width of the serpentine structure of the flexible element 100 of the disclosure may determine the strength limit of the flexible element 100.

The flexible element 100 of the disclosure may be connected from the cerebral cortex to a position penetrating the skull by syringe injection, and for this purpose, the stretchability of the flexible element 100 needs to be 50% or more.

To implement this property, in determining the strength limit of the flexible element 100, the angle may be 100 degrees or more, and the ratio (w/R) of the width of the pattern to the radius of the circular arc may be 0.3 or less. Specifically, the circular arc means a part defined by two points corresponding to a half cycle among the repeating patterns of the serpentine structure (see FIG. 11). Here, w is the width of the pattern, and R is the radius of the circular arc in the serpentine structure. In addition, the angle refers to the angle of the circular arc.

In addition, the porosity affects the bending stiffness of the flexible element 100, and thus is an important factor affecting the injection success rate.

The flexible element 100 of the disclosure may have a porosity of 25 to 60%.

Hereinafter, the disclosure will be described in more detail through Examples and Experimental Examples. However, the following examples and experimental examples are only for specifically illustrating the disclosure, and do not limit the scope of the disclosure. That is, simple modifications or changes of the disclosure can be easily carried out by those skilled in the art to which the disclosure pertains, and all such modifications or changes can be regarded to be included in the scope of the disclosure.

### <Example 1>

A brain signal measurement and stimulation structure having a multi-layer graphene electrode was prepared using a Cu sacrificial layer.

First, single-layer graphene was grown on a copper foil having a thickness of 25 um by chemical vapor deposition (CVD) . A roll of Cu foil (thickness: 25 um and size: 210 x 297 mm², Alfa Aesar Co.) was loaded into a quartz tube, and was then heated to 1000°C under normal pressure. After growing graphene on the Cu foil by supplying a gas mixture (CH₄: H₂ = 8: 20 sccm) containing a carbon source, cooling to room temperature was performed in a short time at a rate of -10°C/s by flowing H₂ while moving the furnace, thereby obtaining a graphene layer grown on the Cu foil.

To make a multi-layer graphene electrode, polymethyl methacrylate (PMMA) was used as a graphene support layer. After synthesizing graphene on the Cu film, PMMA as a support layer was spin-coated, and then the film was caused to float on about 0.1 M (NH4)₂S₂O₈ solution to dissolve the copper catalyst. After removing the copper, another graphene-grown Cu foil was used to lift the PMMA/G film. The above etching and transfer methods were repeated to form a multi-layer film. The PMMA-coated graphene obtained after etching the Cu foil by using an aqueous ammonium persulfate solution was transferred onto another graphene on the Cu foil. Then, the graphene was transferred to a SU-8 epoxy substrate. The transferred graphene was patterned using photolithography and oxygen plasma etching. Nitric acid was used to chemically dope graphene.

### <Experimental Example 1: Measurement of resistance change due to tensile deformation>

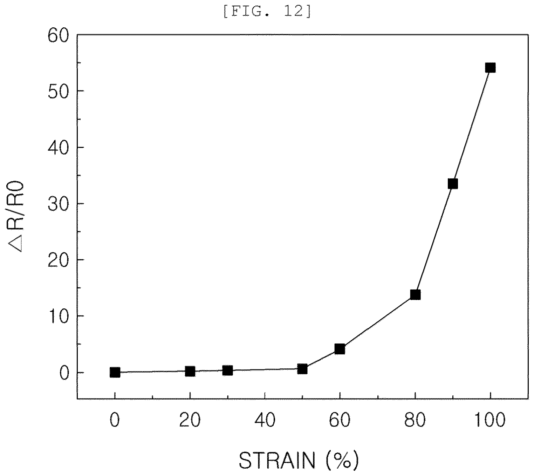

The change in resistance to tensile deformation of the element manufactured in the above Example was measured, and the results are shown in FIG. 11. R represents the resistance measured during tension, and R₀ represents the initial resistance before tension. As noted from FIG. 12, resistance change is less than 0.5% until strain is 50%. Therefore, the element has a very low resistance increase due to tensile deformation and thus may be suitable for the use as the flexible element 100 capable of withstanding deformation occurring during injection.

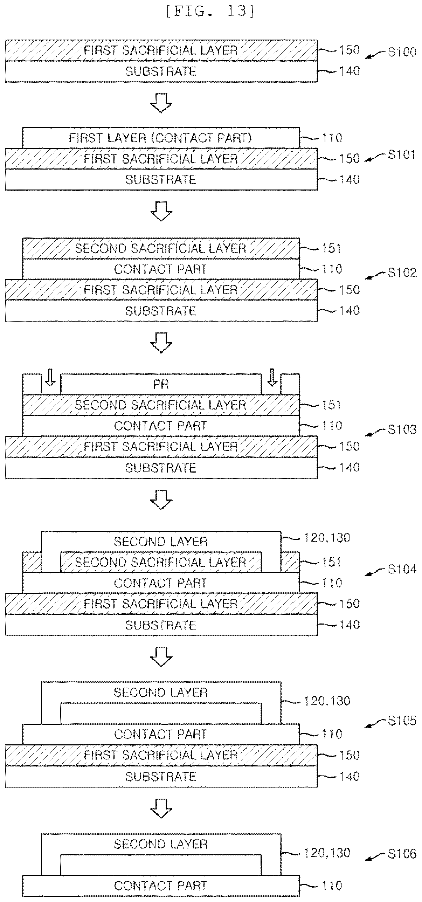

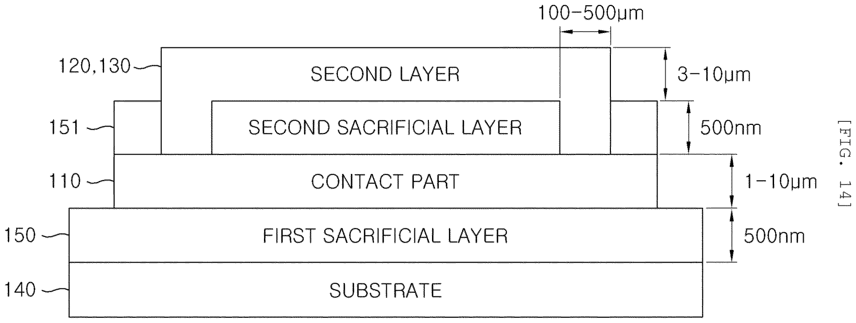

FIG. 13 shows a method for manufacturing a flexible element according to another embodiment of the disclosure, and FIG. 14 shows the thickness and diameter of each layer of a flexible element according to another embodiment of the disclosure.

The flexible element 100 according to another embodiment of the disclosure includes a contact part 110, a connection part 120, and a transmitting/receiving part 130. The flexible element 100 of another embodiment of the disclosure is different only in the shape from those in FIGS. 1 and 2, and identical element thereto. Thus, detailed description is omitted, and the same reference numbers will be assigned to the same elements.

A method for manufacturing the flexible device 100 according to another embodiment of the disclosure includes a first sacrificial layer forming process (S100), a first layer forming process (S101), a second sacrificial layer forming process (S102), a second sacrificial layer opening process (S103), a second layer forming process (S104), a second sacrificial layer removing process (S105), and a first sacrificial layer removing process (S106).

In the first sacrificial layer forming process (S100), a first sacrificial layer 150 is formed on an SiO wafer or a copper substrate 140 by using a deposition process such as thermal evaporation and sputtering.

The first sacrificial layer 150 is formed to have a thickness of 500 nm or more by using oxides that can be wet-etched with materials such as Al₂O₃ or SiO₂.

In the first layer forming process (S101), a contact part 110 made of polyimide is formed on the first sacrificial layer 150 to a thickness of 1 to 10 µm, a pattern is formed by a photolithography process, and the shape of the contact part 110 is formed by dry etching.

In the second sacrificial layer forming process (S102), a second sacrificial layer 151 is deposited on the contact part 110 by using a deposition process such as atomic layer deposition (ALD), thermal evaporation, or sputtering.

The second sacrificial layer 151 is formed to have a thickness of 500 nm or more by using a metal material such as Ni or Al.

In the second sacrificial layer opening process (S103), a pattern is formed on the second sacrificial layer 151 by a photolithography process, and wet-etching is performed by an etchant such as HNO₃ or FeCl₃ to open a part at which the contact part 110 and the connection part 120 are connected to each other. The open part is formed to have a diameter of 100 to 500 µm.

In the second layer forming process (S104), the transmitting/receiving part 130 and the connection part 120 made of polyimide are formed, a pattern is formed by a photolithography process, and the shapes of the connection part 120 and the transmitting/receiving part 130 are formed by dry etching.

As a result, a partial area of the connection part 120 and a partial area of the contact part 110 are connected.

As shown in FIG. 14, the thickness of the second layers 120 and 130 excluding the thickness of the sacrificial layer 150 may be 3 to 10 µm.

In the second sacrificial layer removing process (S105), the second sacrificial layer 151 is wet-etched by an etchant having an oxidizing power such as nitric acid or FeCl₃ to leave the shape of the connection part 120 and the transmitting/receiving part 130 and to remove the second sacrificial layer 151.

In the first sacrificial layer removing process (S106), the first sacrificial layer 150 is removed by wet-etching using an etchant such as HF, and thus separation from the substrate 140 is achieved.

A material that does not affect the first sacrificial layer 150 formed of oxides is used as an etchant used to remove the second sacrificial layer 151.

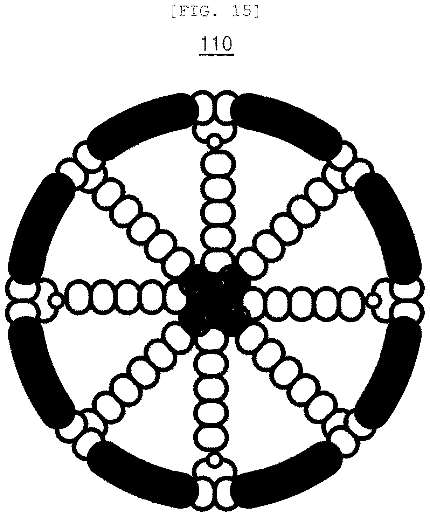

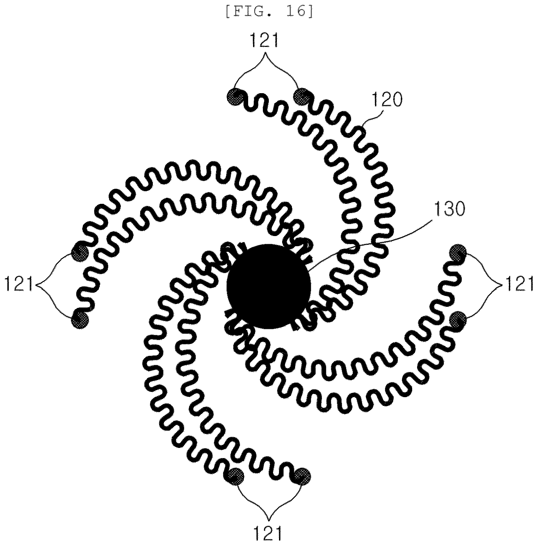

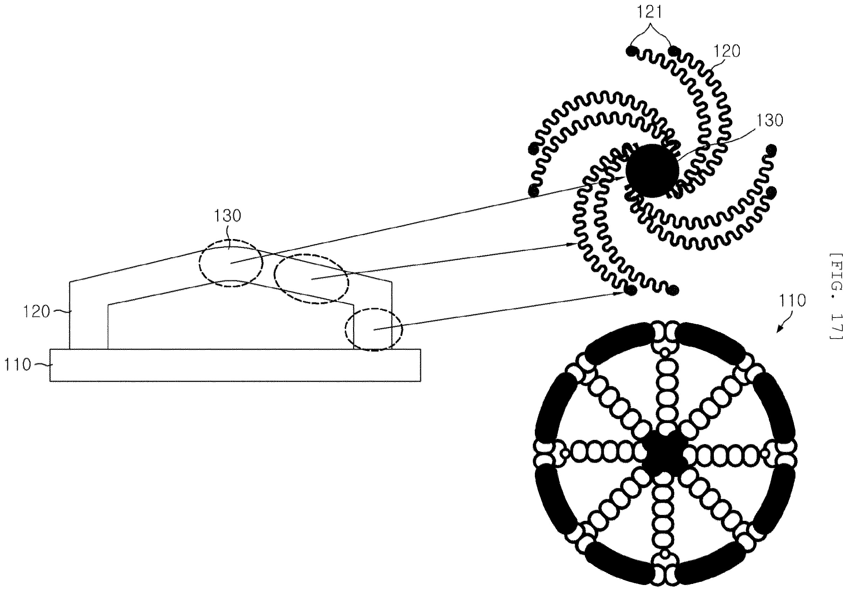

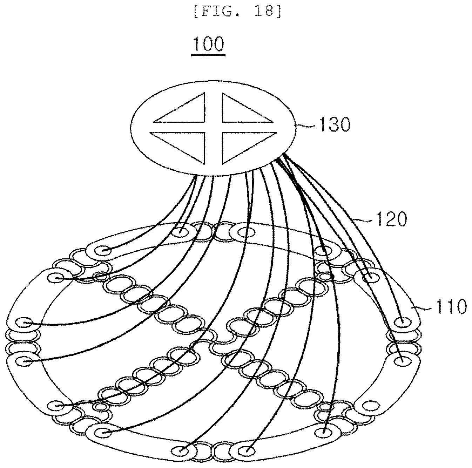

FIG. 15 illustrates a configuration of a contact part of a flexible element according to another embodiment of the disclosure, FIG. 16 illustrates a configuration of a connection part and transmitting/receiving part of a flexible element according to another embodiment of the disclosure, FIG. 17 illustrates a connection relationship between a side view of a flexible element and a plan view of the flexible element according to another embodiment of the disclosure, and FIG. 18 illustrates a spiral shape of a connection part according to another embodiment of the disclosure.

As shown in FIG. 15, the contact part 110 has a central part having a surface shape to receive pressure well, and the size of the central part is appropriately adjusted such that the syringe hole is not blocked by the flexible element 100 when injected into the syringe 13.

The contact part 110 may be connected to an electrode by placing a chip on the surface structure of the central part thereof.

As shown in FIGS. 16 and 17, the connection part 120 may have a coupling area 121, which is to be connected to a partial area of the contact part 110, formed at an end thereof, and may be fabricated into a spirally serpentine shape overall to minimize the influence on the two-dimensional shape of the contact part 110, which is the first layer, and to easily switch to the three-dimensional structure of the connection part 120.

A plurality of connection parts 120 are formed in a serpentine shape, and each of the plurality of connection parts 120 has a spiral shape overall. The circular coupling area 121 formed at an end of each of the connection parts 120 is connected to the edge part of the contact part 110.

When the sacrificial layer 150 is removed in the sacrificial layer removing process (S105), only the coupling areas 121 of the connection parts 120 are connected to each other at the edge part of the contact part 110.

As shown in FIG. 18, when the transmitting/receiving part 130 is lifted, the connection unit 120 is formed in a spiral shape between the contact part 110 and the transmitting/receiving part 130, and thus the flexible element 100 has a three-dimensional structure.

FIG. 19 illustrates a syringe in which a flexible element is accommodated, according to another embodiment of the disclosure, and FIG. 20 shows a structural concept of a flexible element according to another embodiment of the disclosure.

When the flexible element 100 is injected into the brain through the syringe 13, there may also be problems that during the injection process, pressure is not properly applied to the flexible element 100 or is applied to an undesired area, causing the flexible element 100 to be easily crumpled in the skull.

Therefore, the shape of the flexible element 100 needs to be implemented such that the force received from the flow of the liquid is appropriately applied to the flexible element 100.

The injection principle of the syringe 13 is to inject the flexible element 100 by using the pressure and flow of a fluid called cerebrospinal fluid. Since the flexible element 100 has higher chance of being crumpled after injected into the brain when the same has a two-dimensional shape, the flexible element 100 is manufactured in a shape of a three-dimensional structure such that the connection part 120 connects the contact part 110 including a surface shape and the transmitting/receiving part 130 outside the skull, thereby enabling pressure to be applied to a desired location and shape and facilitating easy unfolding after injection (FIG. 17). A method for manufacturing the three-dimensional structure has been described in FIG. 13.

The flexible element 100 injected into the brain by the syringe 13 and unfolded inside the skull may be considered in relation to a structure in which the parachute is unfolded.

The parachute fabric may be the same as the contact part 110 attached to the cerebral cortex by the pressure of the cerebrospinal fluid injected together with the flexible element 100, the line connecting the parachute fabric and a person may be the same as the connection part 120 of the flexible element 100, and the person in the parachute may be considered as a replacement of the transmitting/receiving part 130 of the flexible element 100.

The contact part 110 corresponds to a surface configured to receive the most pressure from the cerebrospinal fluid, and the surface is unfolded by the flow of the cerebrospinal fluid.

The contact part 110 has a surface configured such that, when pressure is applied to the syringe 13 containing the flexible element 100 so as to inject the flexible element 100 into the brain, the surface receives the most pressure from the cerebrospinal fluid included in the syringe 13, and thus is unfolded by the flow of cerebrospinal fluid.

Although the embodiments of the disclosure have been described in detail above, the scope of the disclosure is not limited thereto, and various modifications and improvements by those skilled in the art using the basic concept of the disclosure as defined in the following claims are also included in the scope of the disclosure.

### Industrial Applicability

The disclosure is applicable to a brain implantable medical device by applying a structure including a high-performance flexible element capable of minimizing a skull opening when inserted into the brain.

## Claims

1. A brain signal measurement and stimulation structure (10) comprising:
a flexible element (100) comprising a contact part (110) configured to be in contact with the surface of a cerebral cortex to measure a signal generated in the brain or transmit an external stimulus to the brain, a transmitting/receiving part (130) configured to be positioned between a skull and a skin, and a connection part (120) configured to connect the contact part (110) and the transmitting/receiving part (130); and
an integrated circuit (200) connected to the transmitting/receiving part (130) to transmit and receive a signal,
wherein the flexible element (100) comprises a lower-layer support substrate (111), a graphene electrode layer (113) and a wiring layer (114) formed on the lower-layer support substrate (111), and an insulation layer (115) formed on the graphene electrode layer (113) and the wiring layer (114), the insulation layer (115) is etched such that the graphene electrode layer (113) is partially exposed, and a part of the graphene electrode layer (113) and a part of the wiring layer (114) are adjacently connected, wherein at least one of the contact part (110) and connection part (120) has a serpentine structure.

2. The brain signal measurement and stimulation structure (10) of claim 1, wherein a surface of the insulation layer (115) undergoes hydrophilic surface treatment.

3. The brain signal measurement and stimulation structure (10) of claim 1, wherein a partial surface of the lower-layer support substrate (111) undergoes hydrophobic surface treatment.

4. The brain signal measurement and stimulation structure (10) of claim 2, wherein the hydrophilic surface treatment is performed by spin-coating the insulation layer (115), masking the exposed graphene electrode layer (113), and then treating with O₂ or O₃.

5. The brain signal measurement and stimulation structure (10) of claim 3, wherein the hydrophobic surface treatment is performed such that a hydrophobic microstructure comprising a plurality of microprotrusions is formed.

6. The brain signal measurement and stimulation structure (10) of claim 1, wherein the graphene electrode layer (113) comprises one to four layers.

7. The brain signal measurement and stimulation structure (10) of claim 1, wherein the graphene electrode layer (113) has a diameter of 30 to 150 µm.

8. The brain signal measurement and stimulation structure (10) of claim 1, wherein the wiring layer (114) has a thickness of 30 to 60 nm.

9. The brain signal measurement and stimulation structure (10) of claim 1, wherein the flexible element (100) and the integrated circuit (200) are configured to be injected into the brain of an animal other than a human to receive a brain signal, provide the received brain signal to the outside of the body, and apply one stimulus selected from current, voltage, magnetic field, or electric field stimulus to the brain.

## Patentansprüche

1. Gehirnsignalmess- und -stimulationsstruktur (10), umfassend:
ein flexibles Element (100), umfassend einen Kontaktteil (110), der so konfiguriert ist, dass er mit der Oberfläche einer Großhirnrinde in Kontakt ist, um ein Signal zu messen, das im Gehirn erzeugt wird, oder um einen externen Stimulus an das Gehirn zu übertragen, einen Sende-/Empfangsteil (130), der so konfiguriert ist, dass er zwischen einem Schädel und einer Haut positioniert wird, und einen Verbindungsteil (120), der so konfiguriert ist, dass er den Kontaktteil (110) und den Sende-/Empfangsteil (130) verbindet; und
eine integrierte Schaltung (200), die mit dem Sende-/Empfangsteil (130) verbunden ist, um ein Signal zu übertragen und zu empfangen,
wobei das flexible Element (100) ein Unterschicht-Trägersubstrat (111), eine Graphen-Elektrodenschicht (113) und eine Verdrahtungsschicht (114), die auf dem Unterschicht-Trägersubstrat (111) gebildet ist, und eine Isolationsschicht (115) umfasst, die auf der Graphen-Elektrodenschicht (113) und der Verdrahtungsschicht (114) gebildet ist, wobei die Isolationsschicht (115) so geätzt ist, dass die Graphen-Elektrodenschicht (113) teilweise freigelegt ist, und ein Teil der Graphen-Elektrodenschicht (113) und ein Teil der Verdrahtungsschicht (114) angrenzend verbunden sind, wobei mindestens eines von dem Kontaktteil (110) und dem Verbindungsteil (120) eine Serpentinenstruktur aufweist.

2. Gehirnsignalmess- und -stimulationsstruktur (10) nach Anspruch 1, wobei eine Oberfläche der Isolierschicht (115) einer hydrophilen Oberflächenbehandlung unterzogen wird.

3. Gehirnsignalmess- und -stimulationsstruktur (10) nach Anspruch 1, wobei eine Teiloberfläche des Unterschicht-Trägersubstrats (111) einer hydrophoben Oberflächenbehandlung unterzogen wird.

4. Gehirnsignalmess- und -stimulationsstruktur (10) nach Anspruch 2, wobei die hydrophile Oberflächenbehandlung durch Schleuderbeschichtung der Isolationsschicht (115), Maskierung der freigelegten Graphen-Elektrodenschicht (113) und anschließendes Behandeln mit O₂ oder O₃ durchgeführt wird.

5. Gehirnsignalmess- und -stimulationsstruktur (10) nach Anspruch 3, wobei die hydrophobe Oberflächenbehandlung so durchgeführt wird, dass eine hydrophobe Mikrostruktur gebildet wird, die eine Vielzahl von Mikrovorsprüngen umfasst.

6. Gehirnsignalmess- und -stimulationsstruktur (10) nach Anspruch 1, wobei die Graphen-Elektrodenschicht (113) eine bis vier Schichten umfasst.

7. Gehirnsignalmess- und -stimulationsstruktur (10) nach Anspruch 1, wobei die Graphen-Elektrodenschicht (113) einen Durchmesser von 30 bis 150 um aufweist.

8. Gehirnsignalmess- und -stimulationsstruktur (10) nach Anspruch 1, wobei die Verdrahtungsschicht (114) eine Dicke von 30 bis 60 nm aufweist.

9. Gehirnsignalmess- und -stimulationsstruktur (10) nach Anspruch 1, wobei das flexible Element (100) und die integrierte Schaltung (200) so konfiguriert sind, dass sie in das Gehirn eines Tieres, das kein Mensch ist, injiziert werden können, um ein Gehirnsignal zu empfangen, das empfangene Gehirnsignal an die Außenseite des Körpers bereitzustellen und einen Stimulus, ausgewählt aus Strom, Spannung, Magnetfeld oder elektrischem Feldstimulus, an das Gehirn anzulegen.

## Revendications

1. Structure de mesure et de stimulation de signal cérébral (10) comprenant :
un élément flexible (100) comprenant une partie de contact (110) configurée pour être en contact avec la surface d'un cortex cérébral pour mesurer un signal généré dans le cerveau ou émettre un stimulus externe au cerveau, une partie d'émission/réception (130) configurée pour être positionnée entre un crâne et une peau, et une partie de connexion (120) configurée pour connecter la partie de contact (110) et la partie d'émission/réception (130) ; et
un circuit intégré (200) connecté à la partie d'émission/réception (130) pour émettre et recevoir un signal, dans laquelle l'élément flexible (100) comprend un substrat de support de couche inférieure (111), une couche d'électrode en graphène (113) et une couche de câblage (114) formée sur le substrat de support de couche inférieure (111), et une couche d'isolation (115) formée sur la couche d'électrode en graphène (113) et la couche de câblage (114), la couche d'isolation (115) est gravée de telle sorte que la couche d'électrode en graphène (113) soit partiellement exposée, et une partie de la couche d'électrode en graphène (113) et une partie de la couche de câblage (114) sont connectées de manière adjacente, dans laquelle au moins l'une de la partie de contact (110) et de la partie de connexion (120) présente une structure en serpentin.

2. Structure de mesure et de stimulation de signal cérébral (10) selon la revendication 1, dans laquelle une surface de la couche d'isolation (115) subit un traitement de surface hydrophile.

3. Structure de mesure et de stimulation de signal cérébral (10) selon la revendication 1, dans laquelle une surface partielle du substrat de support de couche inférieure (111) subit un traitement de surface hydrophobe.

4. Structure de mesure et de stimulation de signal cérébral (10) selon la revendication 2, dans laquelle le traitement de surface hydrophile est effectué par revêtement en centrifugation de la couche isolante (115), par masquage de la couche d'électrode en graphène (113) exposée, puis par traitement avec O₂ ou O₃.

5. Structure de mesure et de stimulation de signal cérébral (10) selon la revendication 3, dans laquelle le traitement de surface hydrophobe est effectué de telle sorte qu'une microstructure hydrophobe comprenant une pluralité de microsaillies soit formée.

6. Structure de mesure et de stimulation de signal cérébral (10) selon la revendication 1, dans laquelle la couche d'électrode en graphène (113) comprend une à quatre couches.

7. Structure de mesure et de stimulation de signal cérébral (10) selon la revendication 1, dans laquelle la couche d'électrode en graphène (113) présente un diamètre de 30 à 150 pm.

8. Structure de mesure et de stimulation de signal cérébral (10) selon la revendication 1, dans laquelle la couche de câblage (114) présente une épaisseur de 30 à 60 nm.

9. Structure de mesure et de stimulation de signal cérébral (10) selon la revendication 1, dans laquelle l'élément flexible (100) et le circuit intégré (200) sont configurés pour être injectés dans le cerveau d'un animal autre qu'un être humain pour recevoir un signal cérébral, fournir le signal cérébral reçu à l'extérieur du corps, et appliquer un stimulus choisi parmi un stimulus de courant, de tension, de champ magnétique ou de champ électrique au cerveau.
